# EUROPEAN PATENT APPLICATION

(11) **EP 1 066 839 A1**
(43) Date of publication of application: **10.01.2001**
(21) Application number: 00305717.1
(22) Date of filing: 06.07.2000
(51) Int. Cl.: A61L 29/08

(54) **Oxidation-resistant endoscope coatings**

(30) Priority: 08.07.1999 GB 9915932
(71) Applicant: Sterilox Medical (Europe) Limited, Abingdon, Oxfordshire OX14 4RY (GB)
(72) Inventor: Muir, Andrew Victor Graham, Guildford, Surrey GU2 5NE (GB); Cross, David, Littlehampton, West Sussex BN16 3AP (GB)
(74) Representative: Caldwell, Judith Margaret

(57) **Abstract**

A polycarbonate-based polyurethane material is used for coating endoscopes and other articles adapted for insertion into and removal for re-use from the human or animal body, the polyurethane material being resistant to oxidising sterilising or disinfecting solutions and is also flexible and biocompatible.

## Description

The present invention relates to sterilisation- or disinfection-resistant coatings for endoscopes and similar medical apparatus which require sterilisation or disinfection between uses.

Endoscopes and other reusable medical devices designed for insertion into the human or animal body generally require sterilisation or disinfection between uses. Traditionally, sterilisation or disinfection of medical equipment is achieved by autoclaving, i.e. by exposing the equipment to steam at high temperature and pressure. While this method is effective, it is often unsuitable for complex and relatively delicate apparatus such as endoscopes which may include components which are not heat-resistant. In order to solve this problem, it has been proposed to use organic sterilising solutions such as glutaraldehyde at ambient temperatures. However, glutaraldehyde has limited sterilising activity, especially in relation to bacterial spores, and can also cause allergic reactions in patients.

A recent improvement has been the use of sterilising solutions which are oxidising in nature, such as peracetic acid and chlorine dioxide, among others. A number of recently-developed oxidising sterilising solutions may be produced, for example, by way of electrochemical processing of aqueous salt solutions, as disclosed for example in GB 2316090. These solutions, such as Sterilox^{™}, are produced by electrochemical cells comprising an anode chamber and a cathode chamber separated by a permeable membrane, and include oxidised chlorine species such as hypochlorous acid as sterilising agents. Such solutions are highly effective in their sterilising action, but are more aggressive than, for example, glutaraldehyde and can attack the plastics and similar components which are used in the construction of endoscopes and the like. The mechanism of degradation is unknown but may involve hydrolysis and/or oxidation.

Currently, the flexible sections of some endoscopes and the like are typically provided with polyurethane-based coatings derived from diols such as polyester macroglycols or polyether macroglycols. A polyurethane is typically the product of three different monomers: polyisocyanate, macroglycol and chain extender. The macroglycol imparts softness/elasticity and contributes to the lubricant and biostability properties of the polyurethane, while the isocyanate and chain extender help to provide hardness. The macroglycol largely dictates the physical and chemical properties of the polyurethane. The coating is typically applied to the endoscope to provide a protective layer and also to improve the aesthetic appearance of the device. However, it has been found by the present Applicant that these polyester- or polyether-based polyurethanes are not sufficiently resistant to degradation by oxidising chemicals used as disinfectants and sterilants.

It is known from US 5,876,331 to provide an endoscope having an external polyurethane coating and an internal vapour barrier layer between the external coating and the inner components, e.g. optical fibres, of the endoscope. The endoscope may be sterilised by exposure to oxidising sterilants in the vapour phase, such as hydrogen peroxide, with the internal vapour barrier layer serving to keep the sterilising vapour away from lubricants which may be provided for the inner components of the endoscope, since chemical reaction therebetween may otherwise form acids which would attack the polyurethane outer coating.

JP 9-305814 also describes an oxidation-resistant coating applied to the outer surface of the insertable flexible tube of an endoscope. The coating is a urethane-silicone co-polymer formed by applying and hardening a coating composition comprising a mixture of a silicone resin and at least one of an aliphatic urethane polymer and an aromatic urethane polymer. However, the resistance of this material to hypochlorous acid-based disinfectants has not been found to be adequate.

According to a first aspect of the present invention, there is provided a method of protecting an article adapted to be inserted into and removed for re-use from a human or animal body against an oxidising sterilising or disinfecting solution by applying a coating of a polycarbonate-based polyurethane material to the article.

According to a second aspect of the present invention, there is provided the use of a polycarbonate-based polyurethane material as an oxidation-resistant coating for an article adapted to be inserted into and removed for re-use from a human or animal body.

According to a third aspect of the present invention, there is provided an article adapted to be inserted into and removed for re-use from a human or animal body characterised in that the article is coated with an oxidation-resistant polycarbonate-based polyurethane material.

According to a fourth aspect of the present invention, there is provided a polycarbonate-based polyurethane material for coating an article adapted to be inserted into and removed for re-use from a human or animal body so as to render the article resistant to an oxidising sterilising or disinfecting solution.

It has been found by the present Applicant that polyurethane materials based on polycarbonate macroglycols are much more resistant to degradation by oxidising chemicals used as disinfectants and sterilants than the known polyether- and polyester-based polyurethanes. Furthermore, the present Applicant has found that polycarbonate-based polyurethanes, if appropriately formulated, have sufficient flexibility that they do not significantly alter the mechanical properties of a device such as an endoscope when coated thereon and do not cause adverse responses in contact with tissue (that is, they are biocompatible).

It should also be noted that the present invention is directed specifically towards endoscopes and other re-usable articles which are intended to be inserted temporarily, e.g. for up to 24 hours, into the human or animal body and then removed, as opposed to devices such as replacement heart valves and the like which are designed for permanent or long-term implantation. Because endoscopes and the like are used more than once and will generally be used on different patients, it is vital that they be sterilised between uses. It will be appreciated that the coating need not be applied to the whole article in question but merely to the or each part which is to be inserted into the body and therefore requires sterilisation or disinfection before and cleaning after each use.

Polycarbonate-based polyurethanes are commercially available, examples being Chronoflex AR™ from CardioTech International (Woburn MA, USA), which includes an aromatic diisocyanate; Chronothane^{™}, also from CardioTech; CarboSil™ and BioSpan-C^{™} (a polycarbonate polyurethaneurea) from Polymer Technology Group Inc. (Berkley CA, USA); Corethane™, Bionate™ and Coremer^{™}, all from Corvita (USA) or from Polymer Technology Group Inc.; and Carbothane^{™} from Thermedics Inc. (Woburn MA, USA). Typically, these polyurethanes are used for long-term implanted medical devices, such as pacemaker leads and artificial heart diaphragms.

A typical example of a polycarbonate-based polyurethane includes poly(1,6-hexyl-1,2-ethylcarbonate)diol as the polycarbonate macroglycol, with the "hard" segment being formed by either an aromatic diisocyanate, such as methylenediisocyanate (MDI), or an aliphatic diisocyanate, such as 4,4-methylenebiscyclohexyldiisocyanate (HMDI). A common chain extender is 1,4-butanediol.

The polycarbonate-based polyurethanes described above are soluble, high molecular weight polyurethanes which can be cast from solution. It is also possible to form the polyurethane *in situ* by mixing the isocyanate component and the macroglycol immediately before being applied as a coating to a substrate. If at least one of these components having a functionality greater than two, then the coating "cures" on the substrate by chemical reaction and is no longer soluble. However, care must be taken when using free isocyanates since these are highly toxic.

Advantageously, the polycarbonate-based polyurethanes may be surface-modified further to improve their resistance to hydrolytic degradation and/or resistance to oxidation, or to introduce other properties. For example, the polycarbonate-based polyurethanes may be modified by the incorporation of surface-active oligomers to tailor the surface chemistry of the base polymer without negatively affecting its bulk properties. Modification may be achieved by after-treating the base polymer with the surface-modifying additive or, more preferably, by bonding to the termini of the base polymer during synthesis. End groups such as silicone, fluorocarbon, hydrocarbon and polyethyleneoxide may carried by the oligomers, with fluorocarbon being especially preferred for providing improved oxidation resistance. CarboSil-F™, a fluorinated silicone polycarbonate polyurethane of Polymer Technology Group Inc., is particularly suitable.

The polycarbonate-based polyurethane material may contain additives such as anti-blocking compounds and adhesion promoters, among others.

A particularly preferred polycarbonate-based polyurethane is a copolymer of a polycarbonate diol and a diisocyanate, such as Chronoflex AR™ (which contains an aromatic isocyanate) or Carbothane™.

Advantageously, the polyurethane material is applied to a part to be coated in the form of a solution in solvent, which is applied to the article to be coated by way of a spraying, dipping or other process and allowed to dry so as to form a resilient coating.

A currently preferred solvent is based on dimethylacetamide (DMAC), although other suitable solvents may be used. The DMAc solvent may also include methyl ethyl ketone (MEK) and toluene. A preferred solution composition is:

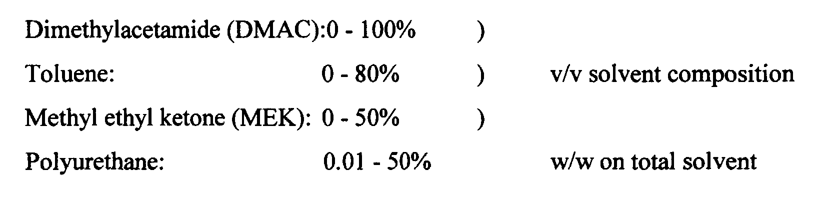

A typical solvent composition is made up in the proportions of 15% DMAC, 35% MEK and 50% toluene.

The MEK and toluene components serve primarily as thinning agents so as to provide an acceptable viscosity for the coating process. In general, a less viscous solution is preferred when spray-coating, and a more viscous solution when dip-coating.

The advantages provided by the polycarbonate-based polyurethane material of the present invention over prior art coating materials are: i) ease of application, ii) good adhesion to the substrate, iii) flexibility without cracking, iv) compatibility with oxidising chemical sterilants and disinfectants such as hypochlorous acid, hydrogen peroxide and peracetic acid as well as conventional sterilising agents such as glutaraldehyde; and v) biocompatibility, i.e. non-toxic and non-sensitising, as opposed to coating materials including free isocyanates.

Of particular importance is the need to provide good resistance to oxidising solutions while being able to withstand stress cracking due to the repeated flexion experienced by endoscopes and the like during normal use.

For a better understanding of the present invention, and to show how it may be carried into effect, a number of exemplary embodiments will now be described.

### Sample preparation

Chronoflex AR/LT^{™}, a polycarbonate-based polyurethane preparation, was obtained from CardioTech International.

The test substrates were, in general, samples of flexible endoscope tubing from three manufacturers: Pentax (Slough, UK), Olympus (Southend, UK), Surgitech (Didam, Holland) and Alternative Endoscopy Instruments (AEI; NJ, USA). Both insertion and light guide tubing were tested. All samples for coating were pre-treated with a degreaser, followed by a solvent to increase adhesion by rendering the surface slightly tacky. Some samples were prepared additionally by using an abrasive paper to increase adhesion of the coating.

A first set of samples (Sample nos. 2 to 8) was dip coated from a 5% solution and dried at elevated temperature. The standard Chronoflex AR™ solution is 100% DMAC, however a solution based on 15% DMAC, 35% MEK and 50% toluene was also evaluated.

A second set of samples (Sample nos. 1 and 9 to 12) were coated by spraying, using a thinner (MEK M6019, Trimite UK) to achieve an acceptable viscosity. Typically, a dilution of the 5% solution by 50% was required, the starting solution being the DMAC/MEK/toluene solution listed above. Two coats were applied and the part was dried for at least 4 hours at 50°C. Further details are given in the table below.

### Polycarbonate-based Polyurethane Coating evaluation

Adhesion of the coating to the tubing surface was assessed by using a fingernail to try and remove the coating. Additionally, a scratch was made in the surface using a sharp scalpel and an attempt made to peel back the coating from scratch. Finally the samples were flexed extensively to determine the effect of adhesion.

| **Sample No.** | **Sample** | **Formulation** | **Comments** |
|---|---|---|---|
| 1 | PVC Tubing | MEK/toluene | Good adhesion |
| 2 | Olympus Insertion Tube | MEK | Good adhesion |
| 3 | Olympus Insertion Tube | MEK | Good adhesion |
| 4 | Olympus Insertion Tube (processed in glutaraldehyde previously) | MEK | Good adhesion |
| 5 | Pentax Light Guide | MEK/DMAC/toluene | Good adhesion |
| 6 | Pentax Insertion Tube | MEK/DMAC/toluene | Good adhesion |
| 7 | Pentax Insertion Tube | MEK/DMAC/toluene | Good adhesion |
| 8 | Pentax Light Guide | MEK/DMAC/toluene | Good adhesion |
| 9 | Pentax Light Guide | MEK/DMAC/toluene | |
| 10 | Olympus Insertion Tube - new tube | MEK/DMAC/toluene | Good adhesion. No speckling. |
| 11 | Surgitech Insertion Tube (small diameter) - new tube | MEK/DMAC/toluene | Good adhesion. No speckling |
| 12 | Surgitech Insertion Tube (large diameter) - new tube | MEK/DMAC/toluene | Good adhesion. No speckling |

Both dip and spray processes give acceptable coating quality. However, trials on complete endoscopes show that a spray process is much more practical, because it is relatively easy to mask areas which are not required to be coated, such as the distal tip (bending section) and the control mechanism. The use of MEK thinners was found to cause the least damage to the surface of typical flexible endoscope bending components whilst giving sufficient adhesion. A formulation of Chronoflex AR^{™} in a solvent comprising 15% DMAC, 35% MEK and 50% toluene was found to be better than a standard formulation in 100% DMAC.

However, due to the variable nature of endoscope substrates (manufacturers can change the identity of the materials at any stage over the product lifetime), different formulations of the solvent vehicle may be chosen so as to provide good adhesion to a given plastics substrate. If necessary, the pre-treatment of the substrate may be varied according to the expertise of the applicator, to achieve sufficient adhesion.

The ability of the coating to maintain adhesion under elongation was tested by extensively manipulating the coated parts within the guidelines of the manufacturers (that is, that the radius of curvature should not be lower than 12 inches) and inspecting for "rucking" due to differing flexibility between the coating and the substrate. All coated parts showed no change in coating appearance when this procedure was followed.

### Sterilox™ compatibility of Polycarbonate-based Polyurethane Coating

A Sterilox™ solution (hypochlorous acid) was prepared in a 2500 Sterilox™ machine and the compatibility of coated and uncoated parts tested. A washer-disinfector was used to contain the parts under test which were removed for inspection at intervals. The available free chlorine (AFC) and pH of the Sterilox™ solution were monitored daily. In order to maximise the number of cycles, a simple two step programme was used:
- Sterilox™ 5 minutes, plus time to fill/drain; and
- Rinse 2 minutes with Sterilox™ rinse water plus fill/drain time.

Additional testing was performed by the immersion of coated endoscope parts in Sterilox™ fluid for a time (up to 4 days), removing the parts and inspecting them visually.

| **Sample** | **Coating Method** | **Compatibility** |
|---|---|---|
| PVC Tubing | Sprayed onto cold part | Wet/Dry - No change (several hours immersion) |
| Pentax Light Guide | Dip | Wet - No change |
| | | Dry - No change (72 hours immersion) |
| Pentax Insertion Tube | Dip | Wet - Pinpricking/rough texture. |
| | | Dry - Above changes gone. Marks remain on coating only (72 hours immersion) |
| Pentax Insertion Tube | Dip | Slightly lightening in colour |
| Pentax Light Guide | Dip | No Change(90 cyc) |
| Olympus Insertion Tube - new tube | Sprayed onto pre-heated part | 24 hours - no change. |
| | | 120 hours - white in parts but on drying reverts to no change. No tackiness. |
| Surgitech Insertion Tube (small diameter) - new tube | Sprayed onto pre-heated part | 24 hours - no change. |
| | | 120 hours - white in parts but on drying reverts to no change. No tackiness. |
| Surgitech Insertion Tube (large Diameter) - new tube | Sprayed onto pre-heated part | 24 hours - no change. |
| | | 120 hours - white in parts but on drying |
| | | reverts to no change. No tackiness. |

The ability of the coating to maintain adhesion under elongation after exposure to Sterilox™ was tested by extensively manipulating the coated parts within the guidelines of the manufacturers (that is, that the radius of curvature should not be lower than 12 inches) and inspecting for "rucking" due to differing flexibility between the coating and the substrate. All coated parts showed no change in coating appearance.

### Uncoated Control Sample Compatibility

| **Sample** | **Immersion** | **Observations** |
|---|---|---|
| AEI Insertion Tube | 27 cycles | Surface dulls and whitens on flexing |
| AEI Insertion Tube | 67 cycles | Tacky surface |
| Surgitech Insertion Tube | 133 cycles | White surface. Tacky when wet. |
| Surgitech Insertion Tube | 48 hours | White and sticky |
| Pentax Light Guide | 12 hours | White surface |

These results show that a polycarbonate-based polyurethane coating is substantially more compatible with Sterilox™ than the components used by the manufacturers of existing flexible endoscopes. Application of polycarbonate-based polyurethane coatings to the surface of the flexible section of endoscopes appears to eliminate the development of tackiness or whitening. There are some changes in the mechanical properties of the coating, which mean that under extreme challenge (scratching) after prolonged exposure the coating can be damaged. However these condition should not arise during normal use of a flexible endoscope.

In comparison, endoscope flexible sections untreated with polycarbonate-based polyurethane coatings all showed considerable changes on immersion in Sterilox^{™}, some at as little as 27 sterilisation cycles.

### Compatibility of Polycarbonate-based Polyurethane Coating with other Disinfectants

Samples were immersed in the appropriate disinfectant for a period of time shown in the table of results below. They were then removed and inspected for any changes.

| **Test Part** | **NuCidex™ (10 days)** | **Cidex**^{**™**} **(5 days)** |
|---|---|---|
| Pentax Light Guide | Whitening of speckled areas. | Whitening of speckled areas. |
| | No change to remainder of surface. | No change to majority of surface. |

Polycarbonate-based polyurethane coatings are substantially compatible with NuCidex™ and Cidex™. NuCidex™ is a peracetic acid-based sterilising solution which is oxidising, while Cidex™ is a conventional glutaraldehyde-based disinfectant.

### Flexibility

Samples coated as described in the section headed **Sample preparation** were evaluated for their flexibility by bending, by persons experienced in the handling of flexible endoscopes. There was no reduction in flexibility or deterioration in handling properties. This is consistent with the measured mechanical properties of Chronoflex AR™, particularly the 500% elongation by ASTM D-412 for an unstressed sample.

Accordingly, the Applicant has found that polycarbonate-based polyurethane coatings are compatible with the reprocessing of flexible endoscopes because:
- good adhesion of coating to flexible endoscopes can be achieved;
- high flexibility (high elongation and low flexural modulus) of the coating ensures that the adhesion of the coating is maintained when the underlying substrate is manipulated within the limits specified by the manufacturer of the endoscope;
- the coating shows minimal changes on exposure to oxidant sterilants, namely Sterilox^{™}, Cidex^{™} and NuCidex™; and
- the coating is non-cytotoxic, non-sensitising and non-irritating to mucous membranes.

Those skilled in the art will appreciate that the present invention may be embodied in other specific forms without departing from its spirit or essential attributes. Accordingly, reference should be made to the appended claims and other general or conceptual statements herein rather than to the foregoing exemplary embodiments as indicating the scope of the invention.

## Claims

1. A method of protecting an article adapted to be inserted into and removed for re-use from a human or animal body against an oxidising sterilising or disinfecting solution by applying a coating of a polycarbonate-based polyurethane material to the article.

2. A method according to claim 1, wherein the polycarbonate-based polyurethane material is derived from a macroglycol comprising a poly(1,6-hexyl-1,2-ethylcarbonate)diol component.

3. A method according to claim 1 or 2, wherein the polycarbonate-based polyurethane material is derived from an aromatic diisocyanate component.

4. A method according to claim 1, 2 or 3, wherein the polycarbonate-based polyurethane material is derived from an aliphatic diisocyanate component.

5. A method according to any preceding claim, wherein a 1,4-butanediol is used as a chain extender in the polycarbonate-based polyurethane.

6. A method according to any preceding claim, wherein the polycarbonate-based polyurethane is surface modified.

7. A method according to claim 6, wherein the polycarbonate-based polyurethane is modified by one or more surface-active oligomers.

8. A method according to claim 7, wherein the one or more surface-active oligomers comprises a fluorocarbon.

9. A method according to any preceding claim, wherein the polycarbonate-based polyurethane material is applied as a solution.

10. A method according to claim 9, wherein the polycarbonate-based polyurethane material is applied by dipping.

11. A method according to claim 9, wherein the polycarbonate-based polyurethane material is applied by spraying.

12. A method according to any preceding claim, wherein the article is part of an endoscope.

13. The use of a polycarbonate-based polyurethane material as an oxidation-resistant coating for an article adapted to be inserted into and removed for re-use from a human or animal body.

14. The use of a polycarbonate-based polyurethane material as claimed in claim 13, wherein the article is part of an endoscope.

15. An article adapted to be inserted into and removed for re-use from a human or animal body characterised in that the article is coated with an oxidation-resistant polycarbonate-based polyurethane material.

16. An article as claimed in claim 15, wherein the article is part of an endoscope.

17. A polycarbonate-based polyurethane material coated onto an article adapted to be inserted into and removed for re-use from a human or animal body so as to render the article resistant to an oxidising sterilising or disinfecting solution.
